# EUROPEAN PATENT APPLICATION

(11) **EP 2 474 267 A1**
(43) Date of publication of application: **11.07.2012**
(21) Application number: 10813769.6
(22) Date of filing: 02.09.2010
(51) Int. Cl.: A61B 8/12, A61M 25/00

(54) **CATHETER**

(30) Priority: 04.09.2009 JP 2009205143
(71) Applicant: Terumo Kabushiki Kaisha, Tokyo 151-0072 (JP)
(72) Inventor: ISHIGURO, Akifumi, Fujinomiya-shi Shizuoka 418-0015 (JP)
(74) Representative: Dossmann, Gérard
(86) International application number: PCT/JP2010/065019
(87) International publication number: WO 2011/027821

(57) **Abstract**

There is provided a catheter in which it is possible to improve work performance even in case of including a window portion. The catheter 1 of the present invention is **characterized by** including: a sheath 2 which is inserted inside a coelom and which is provided with a window portion 26 through which an inspection wave is passed; a detection unit 41 which is movable toward the axis direction of the sheath 2 inside the sheath 2 and which detects the inspection wave; and a reinforcement tube 4 which can cover the inner surface or the outer surface of the window portion 26 and which is movable toward the axis direction of the sheath 2.

## Description

### TECHNICAL FIELD

The present invention relates to a catheter which is used by being inserted inside a coelom such as a blood vessel, a vascular channel and the like.

### BACKGROUND ART

In case of examining a target lesion inside a coelom such as a blood vessel, a vascular channel and the like, there is used an ultra-sound catheter for transmitting and receiving an ultra-sound at the target lesion. For example, the ultra-sound catheter described in Patent Document 1 includes a transducer unit for transmitting and receiving an ultra-sound, an imaging core provided with a drive shaft for rotating this transducer unit and a sheath for covering this imaging core and concurrently for being inserted inside the coelom. The imaging core is movable in an axis direction inside the sheath. The sheath is formed with an acoustic window portion through which an ultra-sound from the transducer unit is penetratable.

When using the ultra-sound catheter, first, the imaging core is arranged beforehand on the most distal side inside the sheath and concurrently the sheath is carried to a deep portion from the target lesion, and next, while leaving the sheath alone, only the imaging core is continued to back away from the sheath distal end along the acoustic window portion and passed through the target lesion. By making only the imaging core back away, the transducer unit moves by passing through the target lesion from the deep portion, so that it is possible to observe the ultra-sound continuously over a region before and after the target lesion and also to produce a tomographic image of a shape of such as a blood vessel, a vascular channel and the like. Also, it is also possible to carry out reconfirmation of the target lesion and a production of the tomographic image by advancing the imagine core which was once backed away.

However, it is not possible for the acoustic window portion through which the ultra-sound is penetratable to contain a structural body which can reflect the ultra-sound, so that depending on the resin material or the like used for the acoustic window portion, there is sometimes a case in which rigidity is low, pressureability (PRESSUREABILITY), anti-kink property, following-ability or the like is low, and work performance is low.

The present invention was invented in order to solve the problem mentioned above and is addressed to provide a catheter in which it is possible to improve work performance even in case of including a window portion.

### Related-Art Document

### Patent Document

Patent Document 1: Japanese unexamined patent publication No. 2002-360578

### DISCLOSURE OF THE INVENTION

A catheter of the present invention addressed to achieve the object mentioned above is characterized by including: a sheath which is inserted inside a coelom and which is provided with a window portion through which an inspection wave is passed; a detection unit which is movable toward the axis direction of the sheath inside the sheath and which detects the inspection wave; and a reinforcement tube which can cover the inner surface or the outer surface of the window portion and which is movable toward the axis direction of the sheath.

It is possible for the catheter of the present invention, which is constituted as mentioned above, to cover the inner surface or the outer surface of the window portion and there is provided with a reinforcement tube which is movable in the axis direction of the sheath, so that in response to the usage state of the catheter, it is possible to cover the window portion by the reinforcement tube or to expose it. Consequently, for example, when inserting the sheath inside the coelom, it is possible, by covering the window portion with the reinforcement tube, to improve pressureability, anti-kink property and steerability such as following-ability, and it is possible to pass it through excellently until the aimed position. Also, when carrying out observation by a detection unit, it is possible, by moving the reinforcement tube and by exposing the window portion, to carry out the observation through the window portion having high permeability for the inspection wave.

If it is constituted such that there is included a reinforcement tube steering unit for moving the reinforcement tube toward the axis direction of the sheath, it is possible to move the reinforcement tube freely by the reinforcement tube steering unit and it is possible to improve work performance.

If it is constituted such that the reinforcement tube steering unit includes a case main body attached to the sheath; and a rotation member for which it is possible to rotate manually by being interlinked rotationally inside the case main body and also which moves the reinforcement tube by a rotation force thereof, it is possible, by rotating the rotation member manually, to move the reinforcement tube and it is possible to improve work performance.

If it is constituted with respect to the reinforcement tube and the detection unit such that the relative position thereof to the axis direction of the sheath is fixedly movable, and the end portion on the sheath distal side of the reinforcement tube is positioned at the sheath proximal portion of the detection unit, it is possible, while exposing the window portion in the region ant which the detection unit is positioned always in an observable state, to reinforce the window portion on the sheath proximal side away from the detection unit by the reinforcement tube. Also, the reinforcement tube and the detection unit are moved simultaneously, so that it is not necessary to steer the reinforcement tube and the detection unit separately and the work performance thereof is excellent.

If it is constituted such that there are included a drive shaft which is inserted inside the sheath and at which the detection unit is fixed on the sheath distal side thereof for transmitting a mechanical driving force; and a hub for moving the drive shaft to the axis direction of the sheath by moving the drive shaft while holding it, wherein the reinforcement tube is interlinked to the hub and is movable toward the axis direction of the sheath together with the hub and the drive shaft, it is possible, by moving the hub, to move the detection unit and the reinforcement tube simultaneously and the work performance thereof is excellent.

If it is constituted such that the sheath includes a housing unit which can accommodate the detection unit on the sheath distal side away from the window portion, it is possible, by housing the detection unit in the housing unit, to cover the window portion completely with the reinforcement tube positioned on the sheath proximal side away from the detection unit and the whole window portion can be reinforced.

### BRIEF DESCRIPTION OF DRAWING

FIG. 1 is a plan view showing an ultra-sound catheter of the present invention;
FIG. 2 is a cross-sectional view in a longitudinal direction showing a junction portion between a distal portion and a main body unit of the ultra-sound catheter;
FIG. 3 is a plan view showing an ultra-sound catheter when moving a reinforcement tube toward the distal side maximally;
FIG. 4 is a plan view showing an ultra-sound catheter when moving a reinforcement tube toward the proximal side maximally;
FIG. 5 is a plan view showing an ultra-sound catheter when pressing an inner tube maximally with respect to a unit connector;
FIG. 6 is a plan view showing an ultra-sound catheter when pulling out an inner tube maximally from a unit connector;
FIG. 7 is a cross-sectional view in a longitudinal direction of a hub;
TIG. 8 is a cross-sectional view in a longitudinal direction of a unit connector and a relay connector;
FIG. 9 is a cross-sectional view in a longitudinal direction of the relay connector;
FIG. 10 is a conceptional diagram showing a relation between an ultra-sound catheter and an external drive apparatus;
FIG. 11 is a plan view showing an ultra-sound catheter in a second exemplified embodiment;
FIG. 12 is a plan view showing an ultra-sound catheter in a third exemplified embodiment;
FIG. 13 is a cross-sectional view in a longitudinal direction of a unit connector and a relay connector when pulling an inner tube of the ultra-sound catheter in the third exemplified embodiment maximally;
FIG. 14 is a cross-sectional view in a longitudinal direction showing a junction portion between a distal portion and a main body unit of the ultra-sound catheter in the third exemplified embodiment; and
FIG. 15 is a cross-sectional view in a longitudinal direction showing the junction portion between the distal portion and the main body unit when moving an imaging core of the ultra-sound catheter in the third exemplified embodiment.

### BEST MODE FOR CARRYING OUT THE INVENTION

Hereinafter, exemplified embodiments of the present invention will be explained with reference to the drawings.

### (First Exemplified Embodiment)

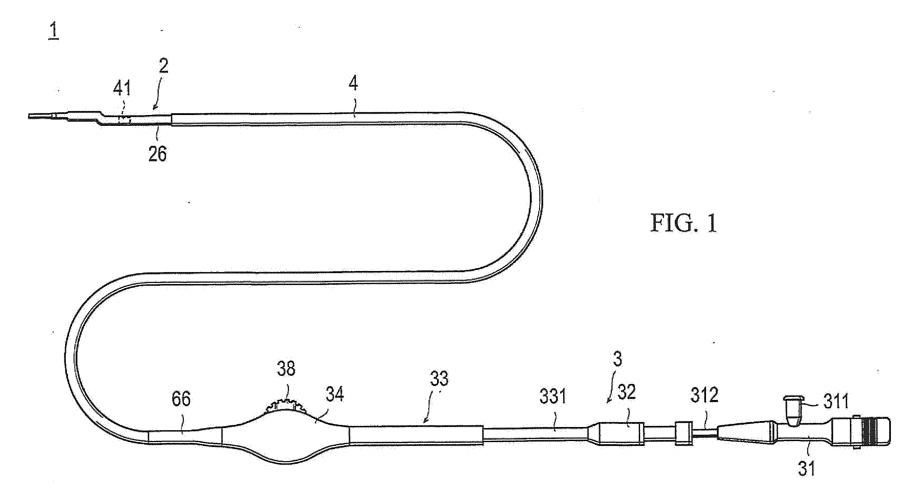

An ultra-sound catheter 1 (catheter) is, as shown in FIG. 1, constituted by a sheath 2 inserted inside a coelom, a reinforcement tube 4 for reinforcing the sheath 2 and a steering unit 3 which is not inserted inside the coelom and arranged on the user's hand side in order to be steered by the user.

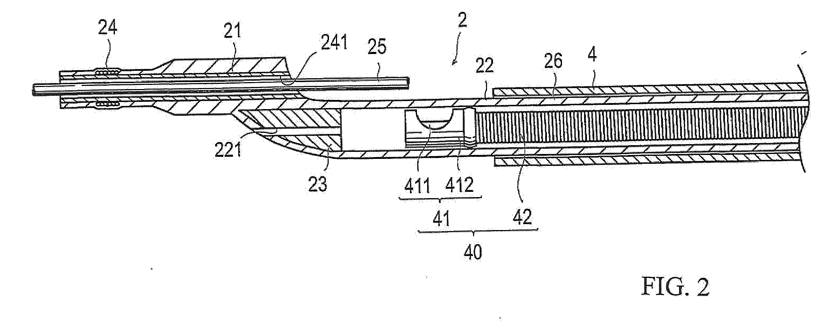

The sheath 2 includes, as shown in FIG. 2, a sheath distal member 21, a sheath main body unit 22 and a filling-liquid in/out-path member 23. The sheath main body unit 22 is bonded with the sheath distal member 21 and the filling-liquid in/out-path member 23 so as to cover the sheath distal member 21 and the filling-liquid in/out-path member 23.

There is provided an X-ray imaging marker 24 between the sheath distal member 21 and the sheath main body unit 22 and it is constituted such that the distal position of the ultra-sound catheter can be confirmed under X-ray fluoroscopy when being inserted inside the coelom.

The sheath distal member 21 is formed with a hole 241 and a guide wire 25 is inserted and passed through this hole 241. The guide wire 25 is inserted inside the coelom beforehand and the ultra-sound catheter 1 is introduced until the target lesion while passing this guide wire 25 through the sheath distal member 21.

Also, the filling-liquid in/out-path member 23 and the sheath main body 22 are formed with a priming lumen 221 which is a hole for flowing out a physiological saline solution filled inside the sheath main body unit 22 to the outside.

The imaging core 40 is installed in the sheath 2 slidably in an axial direction of the sheath 2. This imaging core 40 includes a transducer unit 41 (detection unit) for transmitting and receiving the ultra-sound (inspection wave) toward a tissue inside the coelom and a drive shaft 42 which is attached with this transducer unit 41 at a distal thereof and concurrently which rotates the transducer unit. The transducer unit 41 is constituted by an ultrasonic transducer 411 for transmitting and receiving the ultra-sound and an ultrasonic transducer housing 412 for housing the ultrasonic transducer 411.

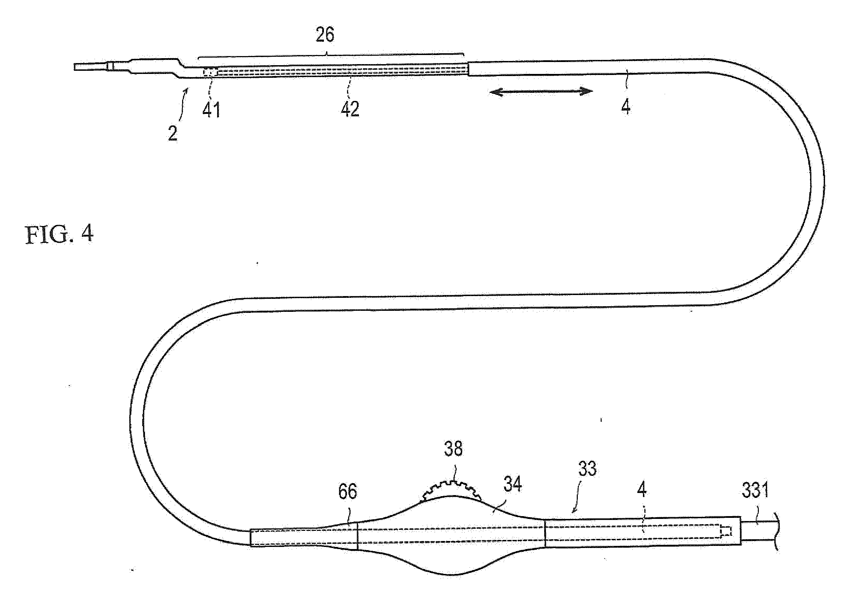

The sheath 2 is formed by a material in which ultra-sounds have high permeability. A site within an area in which the ultrasonic transducer 411 of the sheath 2 moves constitutes an acoustic window portion 26 (window portion) (see FIG. 4). The ultra-sound has a property of being reflected at a boundary portion at which acoustic impedance changes. On an occasion of a diagnosis, more specifically, in a state of indwelling the ultra-sound catheter 1 in a blood vessel, the surrounding area of the ultra-sound catheter 1 is filled with blood (body liquid) Therefore, it is necessary to be constituted such that other than a substance having an equivalent acoustic impedance as that of the blood will not exist between the ultrasonic transducer 411 and a blood vessel wall which is a diagnosis target. It should be noted that the acoustic impendance is a constant peculiar to a material, which is expressed by a product of acoustic speed in the material (speed of sound) and density of the material. In an intraluminal side of the sheath 2, there is injected, as an ultra-sound transmission liquid, with a physiological salt solution whose acoustic impedance approximately coincides with that of the blood. Therefore, it is also necessary for the material constituting the sheath 2 to be a material having an equivalent acoustic impedance. As one example, in this exemplified embodiment, there is used polyethylene for a material of the sheath. 2.

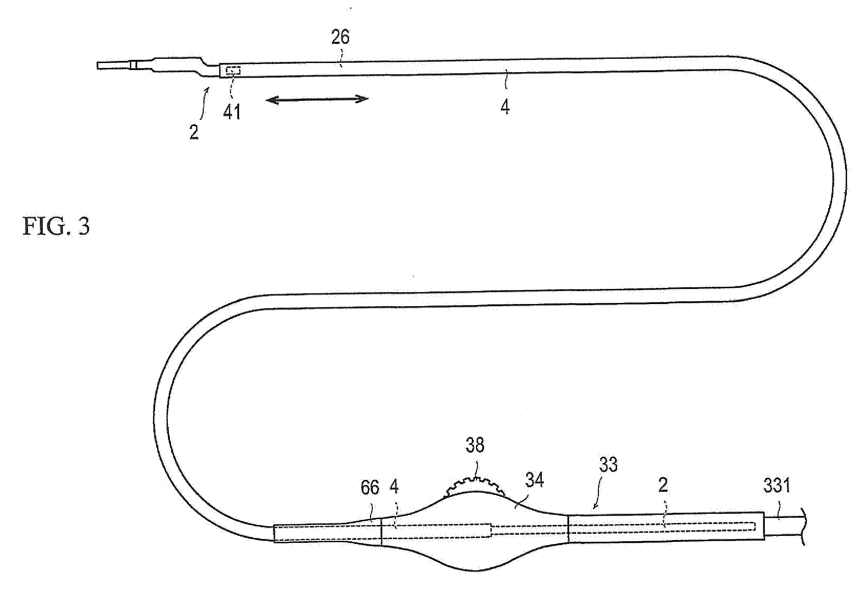

The reinforcement tube 4 is a tubular member for covering the outside of the sheath 2 and as shown in FIG. 3 and FIG. 4, it is provided so as to be movable toward the axis direction with respect to the sheath 2. The reinforcement tube 4 is a member for reinforcing the sheath 2 having low rigidity and it is preferable to be formed by a high strength metal or resin, but if the reinforcement can be achieved, there is no limitation for the material or for the constitution. In this exemplified embodiment, the reinforcement tube 4 is a tubular body made of stainless steel, which is applied with a cut process in a spiral shape.

With respect to the reinforcement tube 4, when being pressed toward the distal side maximally, it is made to be a state of covering the whole of the acoustic window portion 26 (see TIG. 3), and when being pulled-in toward the proximal side maximally, it is made to be a state of exposing the acoustic window portion 26 completely by positioning the distal side on the proximal side of the acoustic window portion 26 of the sheath 2 (see FIG. 4).

The drive shaft 42 shown in FIG. 2 has a characteristic of being flexible and also, in which it is possible for a rotational motion power produced in the steering unit 3 to be transmitted to the transducer unit 41 and, for example, it is constituted by a tube body of a multi-layer coil shape such as a three-layer coil whose winding direction is alternated in a manner from right to left and again right. Owing to a fact that the drive shaft 42 transmits the rotational motion power thereof, the transducer unit 41 rotates and it is possible to observe 360 degrees of the target lesion inside the coelom of such as a blood vessel, a vascular channel and the like. Also, with respect to the drive shaft 42, there is passed through, in the inside thereof, with a signal line for transmitting a signal detected by the transducer unit 41 to the steering unit 3 (see FIG. 1).

The steering unit 3 includes, as shown in FIG. 1, a hub 31 having a port 311 for injecting physiological salt solution for removing air, a unit connector 32 connected with the hub 31 through an inner tube 312 and a relay connector 33 which is connected to the unit connector 32 through the outer tube 331 and concurrently, which connects the sheath 2 and the steering unit 3. The relay connector 33 is provided with a reinforcement tube steering unit 34 for steering the movement of the reinforcement tube 4.

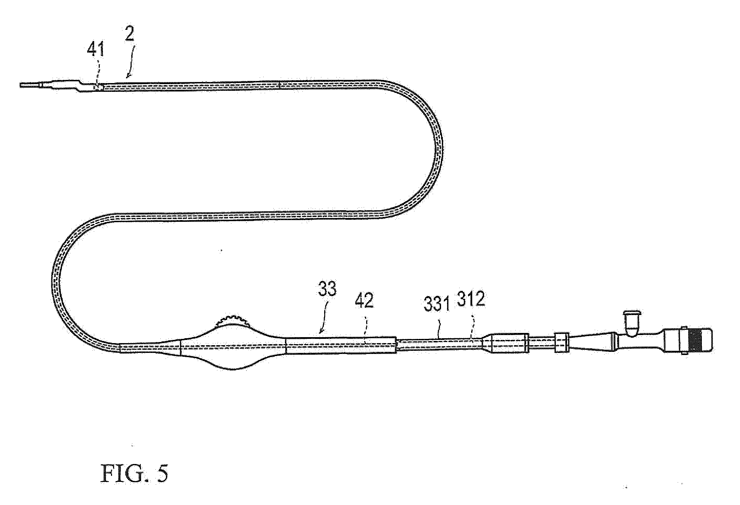

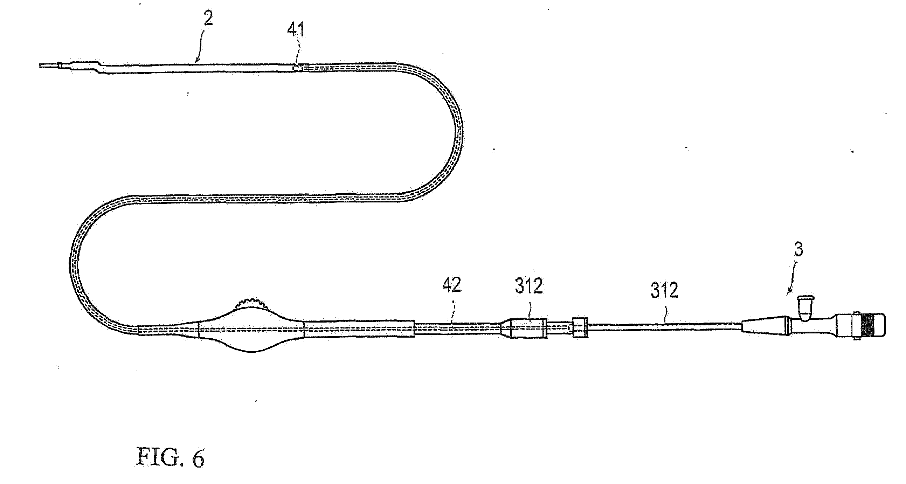

The hub 31 holds the drive shaft 42 and the inner tube 312. By pressing the inner tube 312 into the unit connector 32 and the outer tube 331 or by pulling it out therein, the drive shaft 42 slides cooperatively inside steering unit 3 and the sheath 2 in the axial direction. An aspect of the movement of the drive shaft 42 by the pressing and the pulling-out of the inner tube 312 becomes as shown in FIG. 5 and Fig. 6.

When the inner tube 312 is pressed maximally, as shown in FIG. 5, with respect to the inner tube 312, an end portion thereof on the sheath side reaches until the vicinity of a sheath side end portion of the outer tube 331, more specifically, until the vicinity of the relay connector 33. Then, in this state, the transducer unit 41 is positioned at the vicinity of the distal end of the sheath main body unit 22 of the sheath 2.

Also, when the inner tube 312 is pulled-out maximally, as shown in FIG. 6, with respect to the inner tube 312, a stopper 313 formed at the distal end thereof is engaged with the inner wall of the unit connector 32 and other than the portion which is engaged in the vicinity of the distal end will be exposed. Then, in this state, the transducer unit 41 is pulled back while remaining the sheath 2 in the inside thereof, so that it is positioned at a place toward the side of the steering unit 3 as much as pulling-out the inner tube 312. Owing to a fact that the transducer unit 41 moves while being rotated, it is possible to create a tomographic image of such as a blood vessel, a vascular channel and the like.

Next, a specific constitution of each portion of the ultra-sound catheter 1 will be explained.

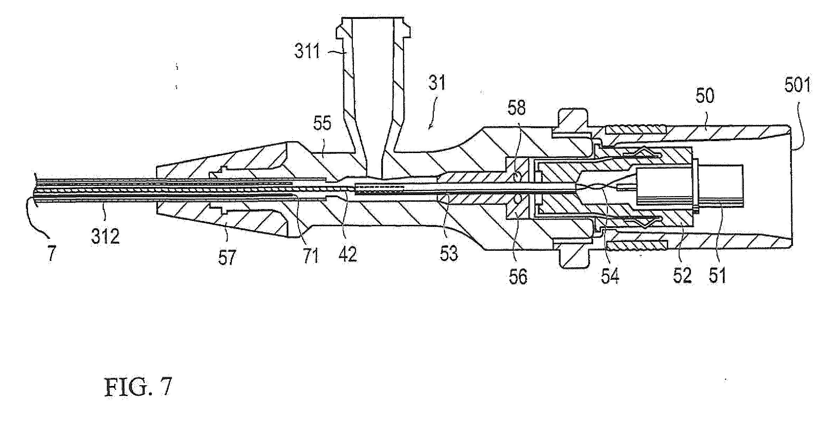

As shown in FIG. 7, the hub 31 includes a joint 50, a male connector 51, a rotor 52, a connection pipe 53, a signal line 54, a hub main body 55, a seal member 56 and an anti-kink protector 57.

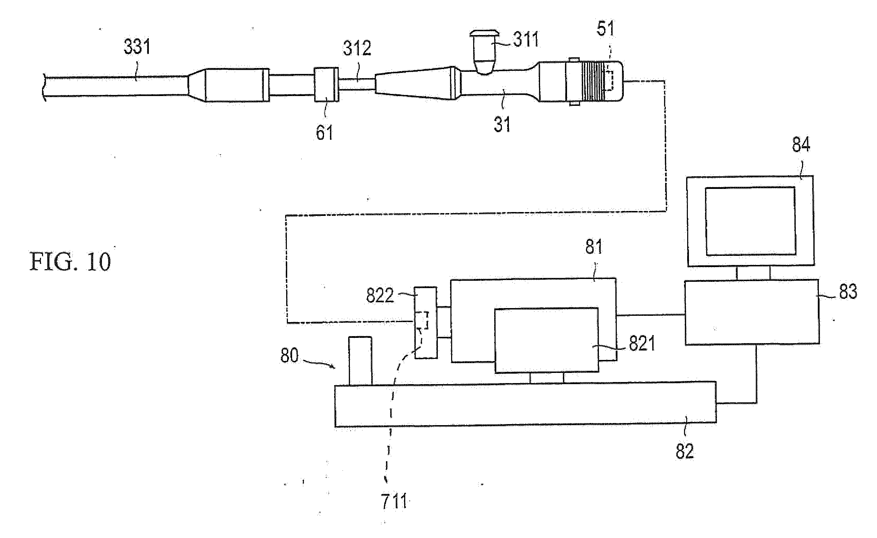

The joint 50 includes an opening portion 501 on the hand-side of a user of the ultra-sound catheter 1 and there are arranged the male connector 51 and the rotor 52 therein. It is possible for the male connector 51 to interlink a female connector 711 which is included in an external drive apparatus 80 (see FIG. 10) from the opening portion 501 side of the joint 50 and thus, mechanical and electrical interlink between the external drive apparatus 80 and the male connector 51 becomes possible.

The external drive apparatus 80 is, as shown in FIG. 10, composed of a scanner device 81 installed with an external drive power supply of a motor or the like, an axial direction moving device 82 which grabs the scanner device 81 and which causes the axial direction movement depending on the motor and the like, a control unit 83 for controlling the scanner device 81 and the axial direction moving device 82 and a display unit 84 for displaying an image obtained by the transducer unit 41. The axial direction moving device 82 includes a scanner device grab portion 821 for grabbing and fixing the scanner device 81 and a sheath support portion 822 for supporting the sheath 2 so as not to be deviated at the time of the pulling-back thereof.

The scanner device 81 carries out transmission & reception of the signal from the transducer unit 41 by being connected to the male connector 51 and simultaneously, transmits driving force for rotating the drive shaft 42.

The ultra-sound scan (SCAN) in the ultra-sound catheter 1, of the present invention is carried out depending on a mechanism of scanning the ultra-sound which is transmitted and received by the ultrasonic transducer 411 provided in the housing 412 in approximately the radial direction by transmitting a rotational motion of the motor in the scanner device 81 to the drive shaft 42 and by rotating the housing 412 fixed at the distal end of the drive shaft 42. The ultra-sound image obtained here is a cross-section image inside the blood vessel. Also, by pulling the whole ultra-sound catheter 1 toward the hand-side and by rendering the imaging core 40 to move in the longitudinal direction, it is possible to obtain a cross-section image of 360° until any desired position by a scanning manner in the surrounding tissues extending over the axial direction inside the blood vessel.

The rotor 52 holds, as shown in FIG. 7, the connection pipe 53 so as not to be rotated and rotates integrally with the male connector 51. The connection pipe 53 holds the drive shaft 42 by an end portion opposite to the rotor 52 side in order to transmit rotation of the rotor 52 to the drive shaft 42. The signal line 54 is passed-through in the inside of the connection pipe 53 and with respect to this signal line 54, one end thereof is connected to the male connector 51, and the other end thereof is passed-through inside the drive shaft 42 and connected to the transducer unit 41. An observation result in the transducer unit 41 is transmitted to the external drive apparatus 80 through the male connector 51, is applied with an appropriate process, and is displayed as an image.

The hub main body 55 is injected with the physiological salt solution from the port 311 and introduces this physiological salt solution into the inner tube 312 without leakage to the outside. It should be noted that there is installed with the seal member 56 including an O-ring 58 between the hub main body 55 and the joint 50, so that it never happens that the physiological salt solution leaks out to the opening portion 501 side of the joint 50.

With respect to the hub main body 55, a portion of the inner tube 312 is fit together by insertion and the anti-kink protector 57 is arranged at the surrounding of the inner tube 312 and the hub main body 55. The anti-kink protector 57 is formed by a material having an intermediate hardness between the hardness of the inner tube 312 and the hardness of the hub main body 55, and it is possible to prevent bending, twisting or the like of the inner tube 312 at the region in which the inner tube 312 exposes from the hub main body 55.

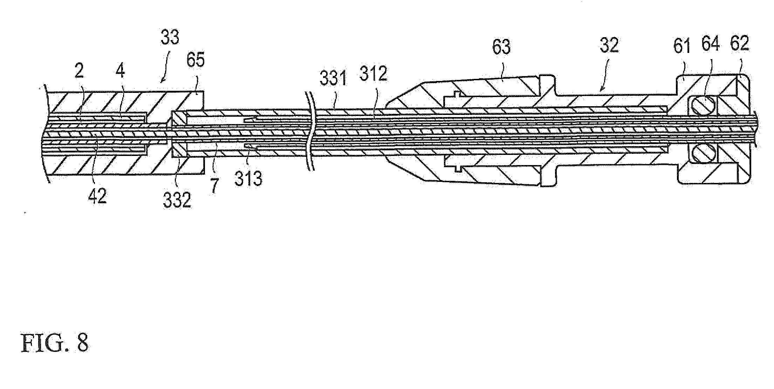

There is arranged inside the inner tube 312 with a protection tube 7 between the drive shaft 42 and the inner tube 312. This protection tube 7 is opened at the end portion on the hub 31 side and includes an end which is not held at all, that is, a free end 71. The protection tube 7 is extended until the outer tube 331 shown in FIG. 8.

The unit connector 32 includes a unit connector main body 61, a sealing member 62, a cover member 63 and a packing 64.

The unit connector main body 61 is inserted with the outer tube 331 attached to the relay connector 33 and the inner tube 312 extended from the hub 31 is inserted in the inside of this outer tube 331. The sealing member 62 holds the packing 64 in combination with the unit connector main body 61 and the cover member 63 holds the outer tube 331 in combination with the unit connector main body 61. The packing 64 is sealed between the, unit connector main body 61 and the sealing member 62, so that even if the physiological salt solution supplied to the port 311 of the hub 31 flows into the outer tube 331 through the inner tube 312, it does not leak to the outside of the unit connector 32.

Also, with respect to the inner tube 312 extending from the hub 31, a stopper 313 is formed at the distal end thereof, so that when pulling the hub 31 maximally, more specifically, even when pulling-out the inner tube 312 from the outer tube 331 maximally, there never happens such a phenomenon that the stopper 313 is engaged with the inner wall of the unit connector main body 61 whereby the inner tube 312 will be pulled out from the unit connector 32.

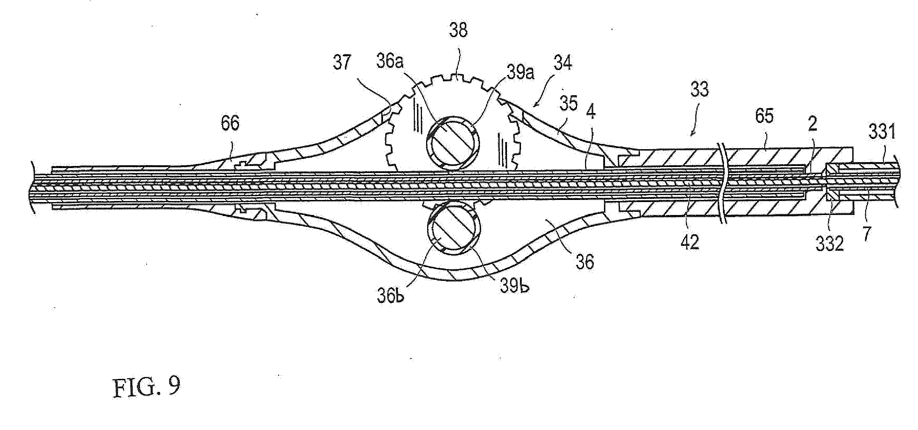

The relay connector 33 includes, as shown in FIGS. 8 and 9, an outer tube hold portion 65, a relay connector main body 66 and a reinforcement tube steering unit 34. The outer tube hold portion 65 holds the outer tube 331. Also, the proximal side end portion of the sheath 2 is interlinked with the inner face of the outer tube hold portion 65, and there is formed a path for introducing the drive shaft 42 passed through from the outer tube 331 and the physiological salt solution into the sheath 2. It is also possible, by inserting a plurality of tubes further into the inside of this path, to prevent buckling of the drive shaft 42, leakage of the physiological salt solution and the like.

A protection tube 7 is fixed on the inner wall of an exit members 332 through which the drive shaft 42 of the outer tube hold portion 65 is passed. This protection tube 7 extends toward the inside of the inner tube 312 extending from the hub 31. Consequently, when the outer tube 331 is pressed into the inner tube 312, it becomes in a state in which the protection tube 7 is pressed into the inner tube 312 in a direction opposite to the direction of the pressing thereof. When the inner tube 312 is pressed or pulled-out with respect to the outer tube 331, it happens that also the protection tube 7 is relatively pressed or pulled-out with respect to the inner tube 312 from the opposite direction, so that even if friction occurs by the contact with the inner tube 312 and a bending force occurs at the drive shaft 42, the bending force is repressed by the protection tube 7, and it is possible to prevent a bending or the like. It should be noted that the protection tube 7 is formed by a loosely wound coil shaped metal tube body and consequently, the physiological salt solution can flow-into from a gap of the coil, so that it never happens that air remains in the outer tube 331.

The reinforcement tube steering unit 34 includes a case main body 35 which is interlinked to the distal side of the outer tube hold portion 65 and in the inside of the case main body 35, there is formed a space portion 36 which passes through the sheath 2, the drive shaft 42 and the reinforcement tube 4. In the space portion 36, there are provided two rotation members 36a, 36b which are interlinked rotationally to the case main body 35 and for one rotation member 36a, there is formed an operation dial 38 coaxially in which a portion of the outer circumferential surface thereof is exposed to the outside from an opening portion 37 provided at the case main body 35. On the outer circumferential surfaces of the two rotation members 36a, 36b, there are fixed high friction members 39a, 39b as slide stoppers which are composed of rubber or the like. The two rotation members 36a, 36b are arranged so as to sandwich the reinforcement tube 4 by the high friction members 39a, 39b. Consequently, by manually rotating the operation dial 38 exposed to the outside, the rotation force of the rotation member 36a is transmitted to the reinforcement tube and it is possible to move the reinforcement tube 4 in the axis direction.

Also, it is allowed for the reinforcement tube steering unit 34 to be provided with a lock mechanism for fixing the rotation member 36a non-rotationally. It is possible for the lock mechanism to employ, for example, a constitution or the like in which the lock mechanism is provided on the case main body 35 slidably such that it can approach to and be spaced from the rotation member 36a or the rotation member 36b, in which caused by the approach, the lock mechanism is engaged with the rotation member 36a or the rotation member 36b for accomplishing the non-rotational fixing.

The relay connector main body 66 is an anti-kink protector which is interlinked on the distal side of the reinforcement tube steering unit 34, and it prevents the bending (kink) of the reinforcement tube 4 and the sheath 2, which is caused by a rapid change of rigidity, while covering and protecting the outer surface of the reinforcement tube 4.

As mentioned above, in the ultra-sound catheter 1 of the present invention, it is possible to press the inner tube 312 into the outer tube 331, so that it is possible for a user to push and pull the hub 31 toward and from the unit connector 32, and it is possible to move the imaging core 40 inside the ultra-sound catheter 1 along the acoustic window portion 26. Then, by rotating the operation dial 38 of the reinforcement tube steering unit 34 manually, the reinforcement tube 4 moves in the axis direction along the sheath 2, so that it is possible to cover the acoustic window portion 26 (see FIG. 3) or to expose it (see TIG. 4) by means of the reinforcement tube 4.

Next, it will be explained with respect to an operation of the ultra-sound catheter 1 of the present invention when observing the inside of a coelom.

Before inserting the sheath 2 of the ultra-sound catheter 1 inside the coelom, there is carried out a priming operation for filling the inside of aforesaid ultra-sound catheter 1 with physiological saline solution. By carrying out this priming operation, it is possible to remove the air inside the ultra-sound catheter 1 and to prevent the air from entering the inside of the coelom of the blood vessel or the like.

With respect to the priming operation, first, the physiological salt solution is injected from the port 311 in a state of pulling the hub 31 maximally toward the hand-side of the user, that is, in a state in which the inner tube 312 is pulled maximally from the outer tube 331. The injected physiological salt solution is to be filled sequentially from the hub 31 to the inside of the sheath 2. When the ultra-wound catheter 1 is filled perfectly with the physiological salt solution, the physiological salt solution will be removed from the priming lumen 221 which is formed in the sheath distal member 21 of the sheath 2. Thus, the filling of the physiological salt solution can be confirmed. It should be noted that it is allowed for the filling of the physiological saline solution to be confirmed by a fact that the physiological saline solution is injected from the priming lumen 221 and the physiological saline solution overflows from the port 311. In addiction, the priming is also necessary between the reinforcement tube 4 and the sheath 2, and by immersing the reinforcement tube 4 into the physiological saline solution, it is possible to carry out the priming between the reinforcement tube 4 and the sheath 2 from a hole of the reinforcement tube 4. Alternatively, it is possible to employ a constitution in which the priming liquid is made to flow out between the reinforcement tube 4 and the sheath 2 from the outer tube hold portion 65 and in which by sealing between the outer surface of the reinforcement tube 4 and the inner surface of the outer tube hold portion 65 by means of a sealing member, it is possible to carry out the priming also between the reinforcement tube 4 and the sheath 2 from the port 311.

Next, as shown in FIG. 10, the ultra-sound catheter 1 is interlinked with the external drive apparatus 80. More specifically, the male connector 51 is interlinked with the female connector of the external drive apparatus 80 and the unit connector main body 61 is interlinked with the sheath support portion 822 of the external drive apparatus 80.

Next, the hub 31 is pressed and there is obtained a state in which the inner tube 312 is pressed maximally in the outer tube 331 and further, by rotating the operation dial 38 manually, the reinforcement tube 4 is moved to the distal side maximally and the acoustic window portion 26 is covered by the reinforcement tube 4 (see FIG. 3). In this state, the sheath 2 is to be inserted into the inside of the body and then, the insertion thereof is stopped after the distal end of the sheath 2 exceeds the target lesion.

At that time, the acoustic window portion 26 having a low rigidity is covered by the reinforcement tube 4, so that steerability of pressureability, anti-kink property, following-ability and the like are excellent and it can be passed through until the aimed position excellently. Also, it is difficult for the acoustic window portion 26 to bend, so that the wire separation in which the guide wire 25 extends toward the direction apart from the sheath 2 is decreased and also, since it is difficult for the acoustic window portion 26 to bend, safety is improve.

Next, the position of the sheath 2 is fixed and by rotating the operation dial 38 manually, the reinforcement tube 4 is moved toward the hand-side and the acoustic window portion 26 is exposed (see FIG. 4). Thereafter, as shown in FIG. 5 and FIG. 6, the transducer unit 41 is made to move in the axis direction while pulling the hub 31 toward the hand-side, and the region extending over the forward and backward portions of the target lesion is observed by the transducer unit 41 through the acoustic window portion 26.

The ultra-sound catheter 1 relating to the first exemplified embodiment includes the acoustic window portion 26 having high permeability of the ultra-sound and at the same time, is provided with the reinforcement tube 4 which can cover the acoustic window portion 26, so that when inserting it inside the coelom, the work performance thereof can be heightened by covering the acoustic window portion 26 having a low rigidity by using the reinforcement tube 4, and on an occasion of observation, it is possible, by moving the reinforcement tube 4 and by exposing the acoustic window portion 26, to carry out the observation through the acoustic window portion 26 having high permeability of the ultra-sound.

### (Second Exemplified Embodiment)

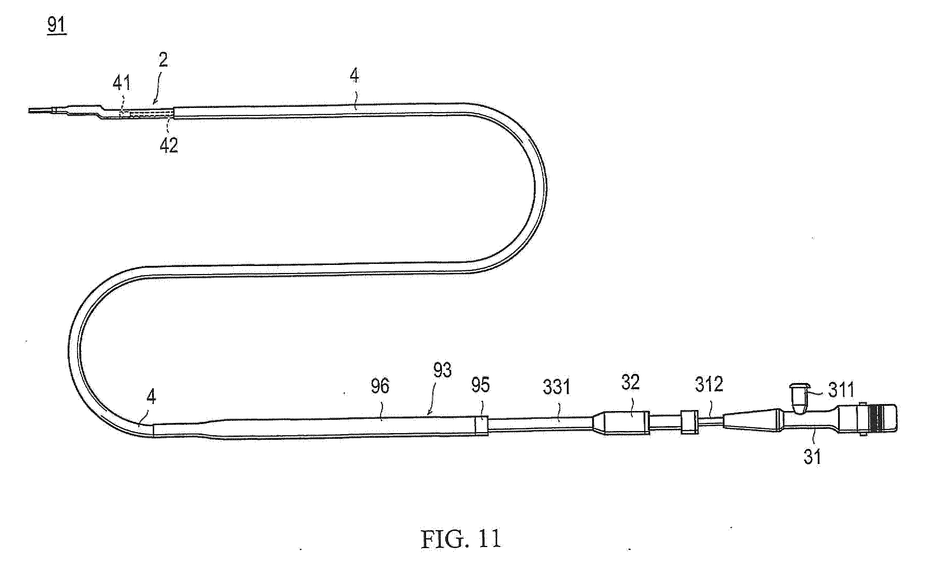

In the first exemplified embodiment, there is provided with a reinforcement tube steering unit 34 for steering movement of the reinforcement tube 4. On the other hand, a ultra-sound catheter 91 relating to the second exemplified embodiment is, as shown in FIG. 11, not provided with a reinforcement tube steering unit. It should be noted in the explanation hereinafter that the same members as those of the first exemplified embodiment are applied with the same reference numerals and the explanation thereof will be omitted.

A relay connector 93 of the ultra-sound catheter 91 includes, as shown in FIG. 11, an outer tube hold portion 95 and a relay connector main body 96.

The outer tube hold portion 95 holds the outer tube 331. Also, the proximal side end portion of the sheath 2 is interlinked with the inner face of the outer tube hold portion 95, and there is formed a path for introducing the drive shaft 42 passed through from the outer tube 331 and the physiological salt solution into the sheath 2.

The relay connector main body 96 is an anti-kink protector which is longer than the relay connector main body 96 of the first exemplified embodiment and which is interlinked on the distal side of the outer tube hold portion 95, and it prevents the bending (kink) of the reinforcement tube 4 and the sheath 2, which is caused by a rapid change of rigidity, while covering and protecting the outer surface of the reinforcement tube 4.

In the ultra-sound catheter 91 relating to such a second exemplified embodiment, it is possible, by directly steering the reinforcement tube 4 which is exposed on the distal side away from the relay connector main body 96, to move the reinforcement tube 4 forward and backward and to open and close the acoustic window portion 26.

### (Third Exemplified Embodiment)

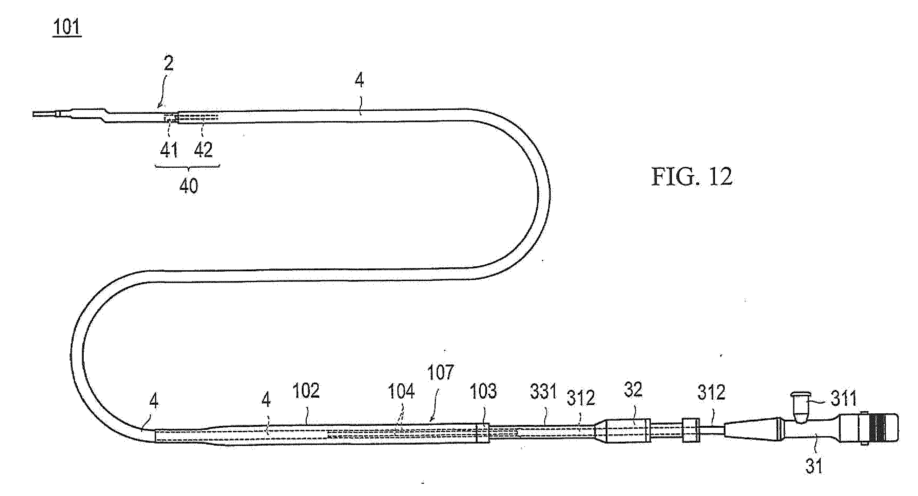

In the first exemplified embodiment, there is provided with a reinforcement tube steering unit 34 for steering the movement of the reinforcement tube 4 and it is possible to steer the forward and backward movement along with the axis direction of the reinforcement tube 4 separately from the imaging core 40. On the other hand, a ultra-sound catheter 101 relating to the third exemplified embodiment is, as shown in FIG. 12, not provided with a reinforcement tube steering unit and the reinforcement tube 4 is moved in synchronization with the imaging core 40 (transducer unit 41). It should be noted in the explanation hereinafter that the same members as those of the first exemplified embodiment are applied with the same reference numerals and the explanation thereof will be omitted.

A relay connector 107 of the ultra-sound catheter 101 includes, as shown in FIG. 12, an outer tube hold portion 103 and a relay connector main body 102.

The outer tube hold portion 103 holds the outer tube 331. Also, the proximal side end portion of the sheath 2 is interlinked with the inner face of the outer tube hold portion 103, and there is formed a path for introducing the drive shaft 42 passed through from the outer tube 331 and the physiological salt solution into the sheath 2.

The relay connector main body 102 is an anti-kink protector which is longer than the relay connector main body 66 of the first exemplified embodiment and which is interlinked on the distal side of the outer tube hold portion 103, and it prevents the bending (kink) of the reinforcement tube 4 and the sheath 2. which is caused by a rapid change of rigidity, while covering protecting the outer surface of the reinforcement tube 4.

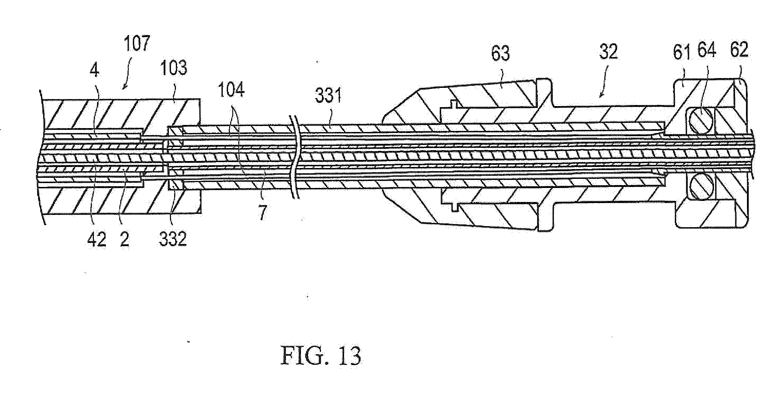

FIG. 13 shows a cross-sectional view in a longitudinal direction of the unit connector 32 and the relay connector 107 when pulling the inner tube 312 maximally. The distal side of the inner tube 312 and the proximal side of the reinforcement tube 4 which are positioned by sandwiching the outer tube hold portion 103 are interlinked, as shown in TIG. 12 and TIG. 13, by an interlink rod 104 passing through a through-hole which is formed at the outer tube hold portion 103. With respect to the interlink rod 104, two pieces thereof are provided in the circumferential direction in this exemplified embodiment, but the number thereof can be other than two pieces. The inner tube 312 moves forward and backward together with the imaging core 40 along with the movement of the hub 31 and therefore, it happens that the reinforcement tube 4 interlinked with the inner tube 312 will move forward and backward together with the imaging core 40.

The distal side of the reinforcement tube 4 is arranged on the proximal side away from the transducer unit 41 of the imaging core 40 in a close relation with the transducer unit 41 as much as possible. Then, the reinforcement tube 4 moves forward and backward together with the imaging core 40, so that it never happens that the reinforcement tube 4 covers the transducer unit 41.

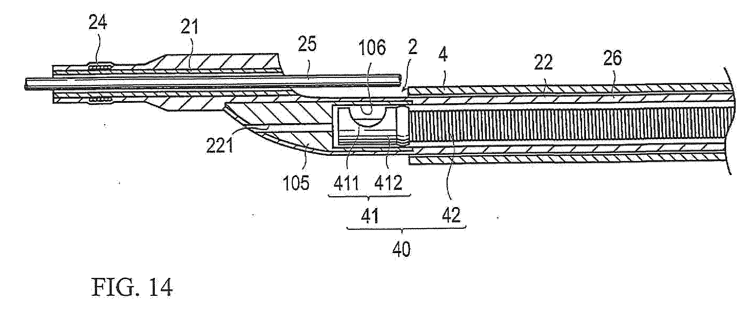

For a filling-liquid in/out-path member 105 which is provided between the sheath distal member 21 and the sheath main body unit 22, as shown in FIG. 14, there is formed a housing unit 106 (housing-unit), which can accommodate the transducer unit 41, in communication with the lumen in the inside of the sheath main body unit 22. The filling-liquid in/out-path member 105 is formed by a material whose rigidity is higher than that of the acoustic window portion 26.

When the transducer unit 41 is housed in the housing unit 106, it becomes in a state in which the reinforcement tube 4 that reaches the proximal side of the transducer unit 41 covers the acoustic window portion 26 completely. More specifically, the reinforcement tube 4 moves forward and backward together with the transducer unit 41, so that it is not possible to cover the transducer unit 41 by the reinforcement tube 4. Consequently, in a case in which it is not possible for the transducer unit 41 to move toward the distal side away from the acoustic window portion 26, it is not possible to cover and reinforce the acoustic window portion 26 completely by means of the reinforcement tube 4. However, in this exemplified embodiment, the transducer unit 41 can be accommodated in the housing unit 106 of the filling-liquid in/out-path member 105 whose rigidity is higher than that of the acoustic window portion 26, so that it is possible to cover the acoustic window portion 26 completely by the reinforcement tube 4 and it is possible to reinforce the whole acoustic window portion 26.

Next, it will be explained with respect to an operation when observing the inside of a coelom by the ultra-sound catheter 101 relating to the third exemplified embodiment.

First, there is carried out a priming operation for filling the inside of the ultra-sound catheter 101 with a physiological saline solution and the ultra-sound catheter 101 is interlinked with the external drive apparatus 80 (see FIG. 10).

Next, the hub 31 is pressed and in a state in which the inner tube 312 is pressed maximally in the outer tube 331 and also in a state in which the transducer unit 41 is housed in the housing unit 106 and the acoustic window portion 26 is covered completely by the reinforcement tube 4 (see FIG. 14), the sheath 2 is to be inserted inside the body and the insertion thereof is stopped after the distal end of the sheath 2 exceeds the target lesion. At that time, the acoustic window portion 26 is covered by the reinforcement tube 4, so that steerability of pressureability, anti-kink property, following-ability and the like are excellent, and it is possible to pass through the sheath until the aimed position excellently. Also, it is difficult for the acoustic window portion 26 to bend, so that the wire separation in which the guide wire 25 extends toward the direction apart from the sheath 2 is decreased and also, since it is difficult for the acoustic window portion 26 to bend, safety is improved.

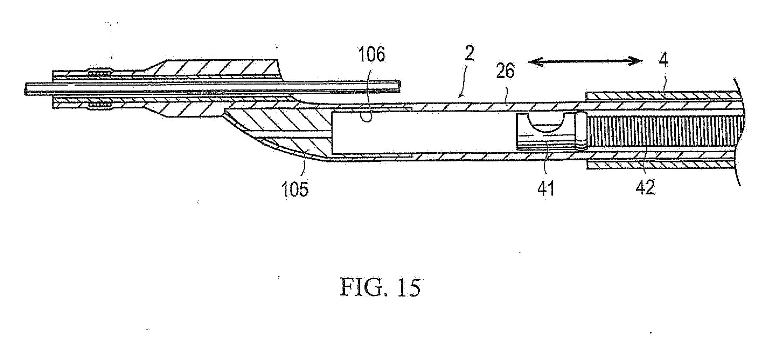

Next, while pulling the hub 31 toward the hand-side, as shown in FIG. 15, the transducer unit 41 is pulled out from the housing unit 106 and the region extending over the forward and backward portions of the target lesion is observed by the transducer unit 41 through the acoustic window portion 26. At that time, the transducer unit 41 moves forward and backward together with the reinforcement tube 4, so that while accomplishing the reinforcement by a configuration in which the acoustic window portion 26 until the proximal side of the transducer unit 41 is always covered by the reinforcement tube 4, it is possible to maintain the exposure of the acoustic window portion 26 in the region in which the transducer unit 41 is arranged. In addition, also the reinforcement tube 4 can be steered simultaneously by steering the hub 31, so that excellent steerability can be exerted even without providing the separate reinforcement tube steering unit 34 such as seen in the first exemplified embodiment.

It should be noted that the present invention is not limited by the exemplified embodiments mentioned above and it is possible to make alteration variously within the scope of claims. For example, in the exemplified embodiment described above, it was explained with respect to a case in which the present invention is applied to an ultra-sound catheter, but it is also possible to apply the present invention to another catheter for diagnosis. For example, it is possible to apply the present invention to a catheter for diagnosis utilizing optical coherence topography (OCT). In the OCT, it is possible to observe a living body by entering a measurement light into the living body and based on the light returning after being scattered or being absorbed, or after being reflected or being refracted inside the living body. Consequently, it is possible for the detection wave to apply not only the ultra-sound but also all kinds of waves which are applicable for the detection of such as light, magnetic field, sound and the like.

Also, in the exemplified embodiment mentioned above, there is used, for the detection unit, a transducer unit for carrying out both the transmission and the reception, but it is also possible to employ a simple constitution in which the detection wave illuminated inside the living body from a position different from that of the catheter is only received (detected) by the detection unit of the catheter.

Also, in the exemplified embodiment mentioned above, the reinforcement tube 4 covers the outside of the acoustic window portion 26, but it is also possible to employ a constitution in which the inside of the acoustic window portion 26 is covered. Also, in order to move the reinforcement tube 4 in conjunction with the imaging core 40 such as shown in the third exemplified embodiment, it is also possible to employ a constitution in which the reinforcement tube is interlinked with the driving power source of the external drive apparatus 80.

Also, in the first exemplified embodiment, there is employed a constitution in which the operation dial 38 is rotated manually, but if a slide member provided on the case main body slidably is to be interlinked to the reinforcement tube 4, it is also possible to move the reinforcement member 4 by moving the slide member forward and backward without using the rotation force.

Further, this patent application is based on Japanese Patent Application No.2009-205143, filed September 4, 2009, and the disclosed contents thereof are hereby incorporated by reference herein in its entirety.

### DESCRIPTION OF REFERENCE NUMERALS

- 1, 91, 101:: ultra-sound catheter (catheter);
- 2:: sheath;
- 4:: reinforcement tube;
- 26:: acoustic window portion (window portion);
- 31:: hub;
- 34:: reinforcement tube steering unit;
- 35:: case main body;
- 36a, 36b:: rotation member;
- 38:: operation dial;
- 41:: transducer unit (detection unit);
- 42:: drive shaft;
- 104:: interlink rod;
- 106:: housing unit.

## Claims

1. A catheter, **characterized by** comprising
a sheath which is inserted inside a coelom and which is provided with a window portion through which an inspection wave is passed;
a detection unit which is movable toward the axis direction of the sheath inside the sheath and which detects the inspection wave; and
a reinforcement tube which can cover the inner surface or the outer surface of the window portion and which is movable toward the axis direction of the sheath.

2. The catheter according to claim 1, **characterized by** comprising a reinforcement tube steering unit for moving the reinforcement tube toward the axis direction of the sheath.

3. The catheter according to claim 2, **characterized in that** the reinforcement tube steering unit includes a case main body attached to the sheath, and a rotation member for which it is possible to rotate manually by being interlinked rotationally inside the case main body and also which moves the reinforcement tube by a rotation force thereof.

4. The catheter according to any one of claims 1 to 3, **characterized in that** with respect to the reinforcement tube and the detection unit, the relative position thereof to the axis direction of the sheath is fixedly movable, and the end portion on the sheath distal side of the reinforcement tube is positioned at the sheath proximal portion of the detection unit.

5. The catheter according to claim 4, **characterized by** further comprising:
a drive shaft which is inserted inside the sheath and at which the detection unit is fixed on the sheath distal side thereof for transmitting a mechanical driving force; and
a hub for moving the drive shaft to the axis direction of the sheath by moving the drive shaft while holding it, wherein
the reinforcement tube is interlinked to the hub and is movable toward the axis direction of the sheath together with the hub and the drive shaft.

6. The catheter according to claim 4 or 5, **characterized in that** the sheath includes a housing unit which can accommodate the detection unit on the sheath distal side away from the window portion.
